(19) **Europäisches Patentamt**

**European Patent Office**

**Office européen des brevets**

(11) Veröffentlichungsnummer: **0 052 745**
**B1**

(12) **EUROPÄISCHE PATENTSCHRIFT**

(45) Veröffentlichungstag der Patentschrift:
16.01.85

(21) Anmeldenummer: 81108265.0

(22) Anmeldetag: 13.10.81

(51) Int. Cl.⁴: **C 07 D 307/89**

(54) **Verfahren zur Herstellung von Phthalsäureanhydrid.**

(30) Priorität: 26.11.80 DE 3044518

(43) Veröffentlichungstag der Anmeldung:
02.06.82 Patentblatt 82/22

(45) Bekanntmachung des Hinweises auf die Patenterteilung:
16.01.85 Patentblatt 85/3

(84) Benannte Vertragsstaaten:
**AT BE DE IT**

(56) Entgegenhaltungen:
**DE - B - 1 193 493**
**FR - A - 2 339 602**
**US - A - 3 466 300**
**US - A - 3 535 345**

(73) Patentinhaber: **BASF Aktiengesellschaft,**
**Carl-Bosch-Strasse 38, D-6700 Ludwigshafen (DE)**

(72) Erfinder: **Stockburger, Dieter, Dr.,**
**Philipp-Krantz-Strasse 14, D-6718 Gruenstadt (DE)**
Erfinder: **Schultz, Wilhelm, Am Wehrhaus 9,**
**D-6718 Gruenstadt (DE)**
Erfinder: **Schmidt, Johannes E., Dr., Pariser Strasse 23,**
**D-6700 Ludwigshafen (DE)**
Erfinder: **Wirth, Friedrich, Dr., Schlossgasse 6,**
**D-6700 Ludwigshafen (DE)**
Erfinder: **Hoffmann, Herwig, Dr., Knietschstrasse 21,**
**D-6710 Frankenthal (DE)**
Erfinder: **Holzknecht, Bernhard, Dr., Haardtstrasse 12,**
**D-6701 Ellerstadt (DE)**
Erfinder: **Wintermantel, Klaus, Dr., Tulpenweg 1,**
**D-6940 Weinheim-Oberflockenbach (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Beschreibung

Die Erfindung betrifft ein Verfahren zur Herstellung von Phthalsäureanhydrid (PSA) durch katalytische Luftoxidation von Naphthalin oder o-Xylol.

PSA wird grosstechnisch durch katalytische Gasphasenoxidation von Naphthalin oder o-Xylol mit Luft hergestellt. Bei diesem bekannten Verfahren wird das o-Xylol in komprimierte und vorgewärmte Luft eingedüst, bzw. die komprimierte und vorgewärmte Luft mit Naphthalindämpfen beladen und das so erhaltene dampfförmige Gemisch, das eine Temperatur von etwa 130 bis 190°C aufweist, zur Oxidation durch einen mit dem Katalysator beschickten Reaktor geleitet. Das den Reaktor verlassende, das PSA enthaltende Reaktionsgas gelangt nach einer Vorkühlung auf Temperaturen von 140 bis 180°C in ein Abscheidersystem, in dem das PSA durch weitere Abkühlung des Reaktionsgases in fester oder flüssiger Form abgeschieden wird.

Je nach dem Gehalt des Kohlenwasserstoffs im dampfförmigen Luftgemisch unterscheidet man zwei Verfahrensvarianten. Bei dem einen Verfahren wird mit einem Betriebsdruck von über 1 bar und mit einem Verhältnis Kohlenwasserstoff/Luft gearbeitet, das meist über der unteren Explosionsgrenze des Kohlenwasserstoff/Luft-Gemisches liegt. Die Oxidation findet hierbei in der Regel im Fliessbett statt und das PSA wird überwiegend flüssig abgeschieden. So wird beispielsweise in der US-Patentschrift 3,535,345 ein Verfahren beschrieben, das im Fliessbett mit 5,2 bis 6,6 bar Betriebsdruck und einem Kohlenwasserstoff/Luft-Verhältnis von 0,1 bis 0,144 betrieben wird und das eine PSA-Flüssigabscheidung von 90% ermöglichen soll. Teile der Kompressionsenergie und fühlbare Wärme im Abgas werden genutzt, indem das Abgas nach Anreicherung mittels Heizgas mit Luft verbrannt wird und die Verbrennungsgase in einer Gasturbine entspannt werden, welche ihrerseits den Prozessluftverdichter antreibt.

Bei dem anderen Verfahren, bei dem es sich meist um ein Festbettverfahren handelt, hält man das Kohlenwasserstoff/Luft-Verhältnis sowohl unterhalb der Explosionsgrenze (für o-Xylol/Luft liegt sie bei 0,044 kg/Nm³) als auch oberhalb der Explosionsgrenze. Dieses Verfahren, das mit einem Betriebsdruck von unter 2 bar betrieben wird, erlaubt keine PSA-Flüssigabscheidung, weil der Taupunkt erst nach Unterschreitung des Schmelzpunktes von PSA erreicht wird. Bei diesem Verfahren wird erheblich weniger Kompressionsenergie benötigt, weil sich die notwendige Druckhöhe der Prozessluft nur an dem Druckverlust der Gesamtanlage orientiert. Andererseits ist die PSA-Abscheidung aufwendig, sie entzog sich auch bisher weitgehend einer zufriedenstellenden Abwärmenutzung. Üblicherweise wird in mehreren parallel betriebenen Abscheidern, die in einem Kühl/Heiz-Zyklus arbeiten, PSA durch Temperatursenkung des Gases fest abgeschieden und nach Umschaltung auf Heizbetrieb durch Abschmelzen flüssig gewonnen. Die Wärmeabfuhr während der Kühlphase erfolgt über ein Wärmeübertragungsmittel, das gegen Umgebungsluft rückgekühlt wird, oder unmittelbar durch das Kühlmedium selbst, z. B. Wasser. Die Nutzung der abzuführenden Wärme ist schwierig, weil das Temperaturprofil des abzukühlenden Gases zu niedrig liegt, um beispielsweise Verdampfungskühlung mit Sattdampferzeugung zu betreiben. Die Prozessluft vor ihrer Verdichtung als Kühlmedium einzusetzen und erwärmt zu verdichten, verbietet der proportional zur absoluten Temperatur zunehmende Kompressionsleistungsbedarf, der ohnehin Hauptanteil des Gesamtenergiebedarfs ist. Nach der Kompression ist die erwärmte Prozessluft zur Kühlung vor allem im Sommerbetrieb nicht mehr geeignet.

Ferner sind als Stand der Technik noch folgende Druckschriften zu nennen:

In der DE-B-1 193 493 wird ein Teil der fühlbaren Wärme des den Reaktor verlassenden Gasgemisches zur Erwärmung der Reaktionspartner genutzt. Wie man den Angaben (s. Spalte 5, Zeilen 41 bis Spalte 6, Zeile 19) entnehmen kann, findet der Wärmeaustausch in den Wärmeaustauschern 12 und 13 statt, wobei die heissen Reaktionsgase von 380°C auf 175°C abgekühlt werden. Mit dieser Temperatur werden die Gase dann in die PSA-Abscheider geleitet, in denen keine weiteren Abwärmenutzung stattfindet.

Aus der US-A-3 466 300 ist bekannt, dass man Anhydride durch katalytische Oxidation der entsprechenden aromatischen Kohlenwasserstoffe mit Luft unter Verwendung eines vielschichtigen Katalysatorbettes herstellen kann. Kennzeichnend ist eine Aufteilung der Gesamtreaktion auf die verschiedenen Schichten, wobei eine maximale Temperaturerhöhung des Gasgemisches durch die Teilreaktion von 50 bis 60°C pro Schicht nicht überschritten wird. Zwischen den Schichten wird die jeweilige Reaktionswärme (nacheinander) an die Einsatzstoffe übertragen, um diese vor ihrem Eintritt in die erste Schicht von 146°C auf 350°C vorzuwärmen (s. Beispiel).

Bei dem in der FR-A-2 339 603 beschriebenen Verfahren wird die Reaktionswärme zur Deckung des Wärmebedarfs der PSA-Destillation unter Verwendung eines organischen Wärmeträgerkreislaufes und zur Erzeugung und Überhitzung von Wasserdampf unter Verwendung eines Salzbadkreislaufes herangezogen. Der überhitzte Dampf wird in einer Kondensationsturbine entspannt, welche ihrerseits den Prozessluftverdichter antreibt.

Es wurde nun gefunden, dass man PSA durch katalytische Luftoxidation von Naphthalin oder o-Xylol, bei der man ein vorgewärmtes Gemisch aus Luft und Naphthalin oder o-Xylol in einem mit dem Katalysator beschickten Reaktor leitet und das den Reaktor verlassende Reaktionsgas durch einen Abscheider führt, in dem das PSA in fester Form abgeschieden wird, besonders vorteilhaft herstellen kann, wenn man die dem Reaktor zuzuführende Luft auf 1,4 bis 1,7 bar komprimiert und nach der Zumischung von Naphthalin oder o-Xylol im PSA-Abscheider durch Abkühlung des Reaktionsgases, das eine Temperatur von 140 bis 180°C aufweist, vorwärmt.

Durch das erfindungsgemässe Verfahren wird die Abwärmeverwertung und der Energiehaushalt bei der grosstechnischen Herstellung von PSA erheblich verbessert und der Investitionsanteil an den Produktkosten durch Wegfall mehrerer Aggregate nebst Zubehör, wie Verrohrung und Instrumentierung deutlich gesenkt.

Nach dem neuen Verfahren wird die Prozessluft, die wie an sich bekannt auf 1,4 bis 1,7 bar komprimiert wird, nach Zumischung des Kohlenwasserstoffes in einem PSA-Abscheider auf Temperaturen von 125 bis 145°C erwärmt.

Bei dem erfindungsgemässen Verfahren wird somit unter wirtschaftlichem Verzicht auf die bisher übliche Erwärmung der Prozessluft und des Kohlenwasserstoffs in separaten Vorerhitzern die Abwärme der PSA-Abscheider für diese Vorerwärmung ausgenutzt.

Das Kohlenwasserstoff/Luft-Gemisch wird nach dieser Vorerwärmung in den Reaktor geleitet. Bei Verwendung von Abscheidern, in denen das PSA in fester Form abgeschieden wird, reicht die Kühlwirkung des Kohlenwasserstoff/Luft-Gemisches zur Abscheidung des PSA in der Regel aus. Sollte beim Betrieb unter hochsommerlichen Temperaturen die Einspritzkühlung der verdichteten Luft durch den Kohlenwasserstoff allein nicht ausreichen, so kann der erwünschte Effekt durch Zugabe eines Kohlenwasserstoff/Wassergemisches erzielt werden, ohne dass dadurch der Taupunkt des Abgases nach dem Abscheider unzulässig erhöht wird.

Für das erfindungsgemässe Verfahren sind zur Abkühlung der den Reaktorbereich verlassenden Reaktionsgase, die eine Temperatur von 140 bis 180°C aufweisen, solche Gemische aus Kohlenwasserstoff und Luft geeignet, bei denen das Kohlenstoff/Luft-Verhältnis unter der unteren Explosionsgrenze liegt. Das sind Gemische, die je $Nm^3$ Luft nicht mehr als 40 g Naphthalin oder 44 g o-Xylol enthalten.

Will man das neue Verfahren auf eine PSA-Herstellung anwenden, bei der ein Gemisch aus Kohlenwasserstoff und Luft in den Reaktor eingeleitet wird, bei dem das Kohlenwasserstoff/Luft-Verhältnis über der unteren Explosionsgrenze liegt, so verfährt man zweckmässigerweise so, dass man den Festabscheider mit einem nichtexplosiblen Gemisch aus Luft und Kohlenwasserstoff beaufschlägt und dieses Gemisch erst unmittelbar vor dem Reaktor durch Zusatz von weiterem Kohenwasserstoff auf das gewünschte Verhältnis aufstockt. Die Übertragung der nutzbaren Wärme aus dem abzukühlenden PSA-haltigen Reaktionsgas auf die Prozessluft kann auch mittels eines zwischengeschalteten Wärmeübertragungssystems, wie z. B. Ölkreislauf, erfolgen.

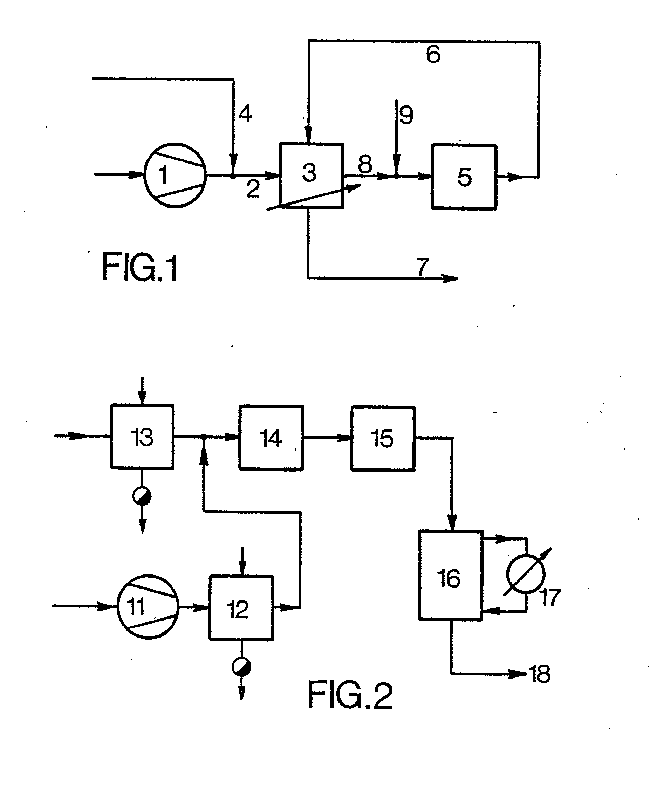

Beispiel 1 (siehe Fig. 1)

56 000 $Nm^3$ Umgebungsluft von 10°C (Jahresmittel) werden pro Stunde in einem Luftverdichter (1) auf 1,513 bar verdichtet. In die verdichtete Luft, die man über eine Zuleitung (2) in den PSA-Abscheider (3) leitet, werden pro Stunde 2240 kg o-Xylol handelsüblicher Qualität und 239 kg Wasser von 25°C eingedüst (4), wobei sich das Gemisch aus Luft, o-Xylol und Wasser auf 32°C abkühlt. Beim Durchströmen des Abscheiders wird das Luft/o-Xylol-Gemisch auf 135°C erwärmt. Dadurch werden dem im Abscheider abzukühlenden Reaktionsgas, das mit einer Temperatur von 170°C aus dem Oxidationsteil (5), der den Reaktor, den Abhitzekessel und den Gaskühler enthält, über die Ableitung (6) in den Abscheider (3) gelangt, pro Stunde eine Nutzwärme von 1,922 Mio kcal entnommen. Die Restwärme wird an die Umgebung abgeführt. Das Abgas verlässt den Abscheider (3) durch die Abteilung (7). Das im Abscheider (3) auf 135°C erwärmte Luft/o-Xylol-Gemisch gelangt über die Leitung (8) in den Reaktor (5). Dabei werden dem Gemisch weitere 2240 kg o-Xylol pro Stunde zugegeben (9).

Im Unterschied zur herkömmlichen Arbeitsweise (siehe Beispiel 2) nutzt man 1,922 Mio kcal/h Abwärme. Dies entspricht bei Verfügbarkeit von 152-grädigem Kesselspeisewasser einer Mehrproduktion von 3,815 to/h 5-bar-Dampf zugunsten des erfindungsgemässen Verfahrens.

Beispiel 2 (Vergleichsbeispiel) (siehe Fig. 2)

56 000 $Nm^3$ Umgebungsluft von 10°C werden pro Stunde in einem Luftverdichter (11) auf 1,513 bar verdichtet und anschliessend in einem dampfbeheizten Wärmeaustauscher (12) auf 130°C vorgewärmt. Gleichzeitig werden pro Stunde 4 480 kg o-Xylol handelsüblicher Qualität in einem dampfbeheizten Wärmeaustauscher (13) auf 150°C erwärmt. Das so vorgewärmte o-Xylol wird mit der vorerwärmten Luft gemischt und das Gemisch in den Oxidationsteil (14), der den Reaktor und den Abhitzekessel enthält, geleitet. Das PSA-haltige Reaktionsgas, das den Oxidationsteil (14) mit einer Temperatur von 370°C verlässt, durchströmt den Gaskühler (15), wo es unter Erzeugung von 6 bar Sattdampf auf ca. 170°C abgekühlt wird. Die restliche abzuführende Wärme wird im PSA-Abscheider (16) an einen Wärmeträgerkreislauf und aus diesem im Luftkühler (17) an die Umgebung abgeführt. Das Abgas verlässt den Abscheider (16) durch die Ableitung (18).

## Patentansprüche

1. Verfahren zur Herstellung von Phthalsäureanhydrid (PSA) durch katalytische Luftoxidation von Naphthalin oder o-Xylol, bei dem man ein vorgewärmtes Gemisch aus Luft und Naphthalin oder o-Xylol in einen mit dem Katalysator beschickten Reaktor leitet und das den Reaktor verlassende Reaktionsgas durch einen Abscheider führt, in dem das PSA in fester Form abgeschieden wird, dadurch gekennzeichnet, dass man die dem Reaktor zuzuführende Luft auf 1,4 bis 1,7 bar komprimiert und nach der Zumischung von Naphthalin oder o-Xylol im PSA-Abscheider durch Abkühlung des Reaktionsgases, das eine Temperatur von 140 bis 180°C aufweist, vorwärmt.

2. Verfahren nach Anspruch 1, dadurch gekennzeichnet, dass der Kohlenwasserstoffgehalt des Gemisches aus Luft und Naphthalin oder o-Xylol, das man im PSA-Abscheider durch Abkühlung des Reaktionsgases erwärmt, unter 40 g Naphthalin oder 44 g o-Xylol je Nm³ Luft liegt.

3. Verfahren nach Anspruch 1, dadurch gekennzeichnet, dass der Wärmeübergang vom Reaktionsgas auf das dem Reaktor zuzuführende Luft/ Kohlenwasserstoffgemisch mit Hilfe eines Wärmeübertragungssystems erfolgt.

## Claims

1. A process for the preparation of phthalic anhydride (PA) by catalytic oxidation of naphthalene or o-xylene with air, a preheated mixture of air and naphthalene or o-xylene being passed into a reactor charged with the catalyst, and the reaction gas leaving the reactor being passed through a separator in which the PA is separated out as a solid, in which process the air fed to the reactor is compressed to 1.4 – 1.7 bar and, after the admixture of naphthalene or of o-xylene, is preheated by using it to cool the reaction gas in the PA separator, the reaction gas having a temperature of from 140 to 180°C.

2. A process as claimed in claim 1, wherein the hydrocarbon content of the mixture of air and naphthalene or o-xylene which is heated in the PA separator by cooling the reaction gas is less than 40 g of naphthalene or 44 g of o-xylene per m³ (S.T.P.) of air.

3. A process as claimed in claim 1, wherein the heat transfer from the reaction gas to the air/ hydrocarbon mixture to be fed to the reactor is effected by means of a heat transfer system.

## Revendications

1. Procédé de préparation de l'anhydride phtalique (AP) par oxydation catalysée du naphtalène ou de l'ortho-xylène par l'air, dans lequel on introduit un mélange d'air et de naphtalène ou d'o-xylène préchauffé dans un réacteur garni d'un catalyseur, puis on fait passer le mélange réactionnel gazeux, quittant le réacteur, à travers un séparateur, dans lequel l'AP est séparé sous forme solide, caractérisé en ce que l'air à introduire dans le réacteur est comprimé entre 1,4 et 1,7 bar et préchauffé après l'addition du naphtalène ou de l'o-xylène, dans le séparateur de l'AP par refroidissement du gaz réactionnel, qui se trouve à une température de 140 à 180°C.

2. Procédé suivant la revendication 1, caractérisé en ce que la teneur en hydrocarbure du mélange d'air et de naphtalène ou d'o-xylène, qui est chauffé dans le séparateur de l'AP par refroidissement du gaz réactionnel, est inférieure à 40 g de naphtalène ou à 44 g d'o-xylène par m³ normal d'air.

3. Procédé suivant la revendication 1, caractérisé en ce que la transmission de la chaleur du gaz réactionnel au mélange d'air et d'hydrocarbure à introduire dans le réacteur est réalisée à l'aide d'un système de transfert de la chaleur.

0052745

FIG.1

FIG.2

5